# EUROPEAN PATENT APPLICATION

(11) **EP 4 711 454 A1**
(43) Date of publication of application: **18.03.2026**
(21) Application number: 24803793.9
(22) Date of filing: 13.05.2024
(51) Int. Cl.: C12N 9/96, C12N 9/18

(54) **COMPOSITION FOR STABILIZING PHOSPHOLIPASE AND METHOD FOR STABILIZING PHOSPHOLIPASE USING SAME**

(30) Priority: 11.05.2023 KR 20230061096
(71) Applicant: CJ Cheiljedang Corporation, Seoul 04560 (KR)
(72) Inventor: LEE, Sungkyun, Seoul 04560 (KR); LEE, Hyo Hyoung, Seoul 04560 (KR); KIM, Taek Beom, Seoul 04560 (KR); KIM, Young Kyung, Seoul 04560 (KR); PARK, Jung Won, Seoul 04560 (KR); KIM, Yu Shin, Seoul 04560 (KR); KIM, Minhoe, Seoul 04560 (KR)
(74) Representative: Meissner Bolte Partnerschaft mbB
(86) International application number: PCT/KR2024/006467
(87) International publication number: WO 2024/232740

(57) **Abstract**

The present disclosure relates to a composition for stabilizing phospholipase and a method for stabilizing phospholipase.

## Description

### [Technical Field]

The present disclosure relates to a composition for stabilizing phospholipase and a method for stabilizing phospholipase.

### [Background Art]

Phospholipase is an enzyme that hydrolyzes phospholipids and is also referred to as lecithinase. Phospholipases are classified into A1, A2, B, C, and D according to the site of cleavage in the phospholipid molecule. Phospholipases A and B produce fatty acids from phospholipids, phospholipase C generates phosphocholine from phospholipids, and phospholipase D separates choline. Among the above phospholipases, phospholipase A2 - is known to cause destruction of cell tissues, hemolytic action, and catalytic reactions as it releases the fatty acid bound to the glycerol of the phospholipid, which is a cell membrane constituent lipid, thereby producing lysophosphatide.

Meanwhile, enzymes contain at least one active site and have an optimal temperature and pH range in which they exhibit maximum catalytic activity as biological catalysts. They possess a sensitive nature in that their activity decreases when conditions deviate from such optimal ranges. In addition, enzymes have a protein structure, and thus can temporarily or permanently lose activity as the structure is sensitively altered by heat or pH. In particular, inactivation of enzymes often occurs during the enzyme production process due to denaturation of the protein during purification and concentration steps, thereby leading to enzyme inactivation, *i.e.*, loss of enzyme activity.

In relation to the above, methods employing additives have been utilized to enhance enzyme stability. For example, Korean Patent Publication No. 1932247 discloses an enzyme composition that maintains stability in both freeze-dried and liquid formulations by containing deoxycholic acid or a salt thereof, as well as a method for stabilizing enzymes using the same.

However, studies on additives capable of preventing enzyme inactivation during purification and concentration steps in the enzyme production process remain limited.

Accordingly, there is still a need for research for preventing inactivation of phospholipase occurring during the manufacturing process.

### [Disclosure]

### [Technical Problem]

An object of the present disclosure is to provide a composition for stabilizing phospholipase, comprising: (i) Tween 20; and (ii) sorbitol, glycerol, or a combination thereof.

Another object of the present disclosure is to provide a method for stabilizing phospholipase, comprising: adding, to a composition comprising phospholipase, a composition for stabilizing phospholipase comprising: (i) Tween 20 and (ii) sorbitol, glycerol, or a combination thereof, to prepare a mixed composition; and concentrating the mixed composition.

Still another object of the present disclosure is to provide the use of a composition comprising: (i) Tween 20 and (ii) sorbitol, glycerol, or a combination thereof, for stabilizing phospholipase.

### [Technical Solution]

The present disclosure provides a composition for stabilizing phospholipase, comprising: (i) Tween 20; and (ii) sorbitol, glycerol, or a combination thereof.

Additionally, the present disclosure provides a method for stabilizing phospholipase, comprising: adding, to a composition comprising phospholipase, a composition for stabilizing phospholipase comprising: (i) Tween 20, and (ii) sorbitol, glycerol, or a combination thereof, to prepare a mixed composition; and concentrating the mixed composition.

Moreover, the present disclosure provides use of a composition comprising: (i) Tween 20, and (ii) sorbitol, glycerol, or a combination thereof, for stabilizing phospholipase.

### [Advantageous Effects]

The composition for stabilizing phospholipase of the present disclosure can solve the problem of enzyme inactivation occurring during the purification and concentration steps in the production of phospholipase, and thus can be usefully applied to the industrial production of phospholipase.

### [Detailed Description of Preferred Embodiments]

The present disclosure will be described in detail as follows. Meanwhile, each description and embodiment described herein can be applied to other descriptions and embodiments, respectively. That is, all combinations of various elements described herein fall within the scope of the present disclosure. Further, the scope of the present disclosure is not limited by the specific description described below.

Furthermore, those skilled in the art may be able to recognize or identify numerous equivalents to the particular aspects of the present disclosure described herein by using routine experimentation. Moreover, these equivalents are intended to be included in the present disclosure.

As used in the specification and appended claims of the present disclosure, the singular articles ("a," "an," and "the") include plural referents unless the context clearly indicates otherwise. Additionally, unless the context indicates otherwise, singular terms include their plural forms, and plural terms include their singular forms. As used in the specification and appended claims of the present disclosure, the use of "or" may include the meaning of "and/or", unless indicated otherwise.

As used herein, terms such as "first, second, third,...", "i), ii), iii),...", or "(a), (b), (c), (d),..." are used to distinguish similar elements and do not imply that the elements are executed continuously or in the listed order. For example, when these terms are used in relation to a method, use, or analytical step, the steps may be performed without any time intervals, simultaneously, or with intervals of several seconds, minutes, hours, days, or months.

As used herein, the term "comprising" refers to the presence of features, steps, or components following the term and does not exclude the presence or addition of one or more other features, steps, or components. As used herein, the components or features following the term "comprising" may be essential or mandatory; however, in certain embodiments, other optional or non-essential components or features may also be included.

An aspect of the present disclosure provides a composition for stabilizing phospholipase, comprising: (i) Tween 20; and (ii) sorbitol, glycerol, or a combination thereof.

Inactivation of enzymes gnenerally occurs during the enzyme production process due to denaturation of the protein during purification and concentration steps, thereby leading to the problem of enzyme inactivation, *i.e.,* loss of enzyme activity. For example, during purification and concentration of the enzyme by ultrafiltration, protein denaturation (aggregation or denaturation) occurs due to shear stress and the like, resulting in enzyme inactivation.

The composition for stabilizing phospholipase of the present disclosure increases the stability of phospholipase by comprising: (i) Tween 20; and (ii) sorbitol, glycerol, or a combination thereof, and thus, it solves the problem of enzyme inactivation during purification and concentration steps in the production of phospholipase, thereby exhibiting a superior effect in increasing product yield and economic feasibility.

In the present disclosure, a standard unit (U) of phospholipase enzyme activity can be defined by the amount of enzyme required to produce 1 µmol of unsaturated polygalacturonic acid per minute by cleaving polygalacturonic acid, but is not limited thereto. Unsaturated galacturonic acid can be used interchangeably with D-galacturonic acid. For example, in the present disclosure, one unit (U) of phospholipase enzyme activity may be defined as the amount of enzyme required to release 1 µmol of unsaturated polygalacturonic acid per minute from a 6 mg/mL polygalacturonic acid solution at 50°C and pH 7.73, but is not limited thereto.

As used herein, the term "phospholipase" refers to an enzyme that hydrolyzes phospholipids. The phospholipase can be used interchangeably with lecithinase. Phospholipases are classified into A1, A2, B, C, and D according to the site of cleavage in the phospholipid molecule. Phospholipases A and B produce fatty acids from phospholipids, phospholipase C generates phosphocholine from phospholipids, and phospholipase D separates choline. Among the above phospholipases, the phospholipase A2 component is known to cause destruction of cell tissues, hemolytic action, and catalytic reactions as it releases the fatty acid bound to the glycerol of the phospholipid, which is a cell membrane constituent lipid, thereby producing lysophosphatide.

In the present disclosure, the phospholipase may be a phospholipase derived from a *Trichoderma* sp. microorganism, but is not limited thereto, and may include any phospholipase without limitation as long as it is stabilized by the composition for stabilizing phospholipase of the present disclosure.

As used herein, the term "Tween 20" is a representative name of commercially available polysorbate 20, which is a nonionic surfactant belonging to the polysorbate series. In the present disclosure, Tween 20 can be used interchangeably with Polysorbate 20.

In one embodiment, the composition for stabilizing phospholipase of the present disclosure may comprise: Tween 20 and sorbitol; Tween 20 and glycerol; or Tween 20, sorbitol, and glycerol.

The composition for stabilizing phospholipase of the present disclosure increases the stability of phospholipase by comprising: Tween 20 and sorbitol; Tween 20 and glycerol; or Tween 20, sorbitol, and glycerol. Therefore, it solves the problem of enzyme inactivation during purification and concentration steps in the production of phospholipase, thereby exhibiting a superior effect in increasing product yield and economic feasibility.

Specifically, the Tween 20 may be included in an amount of 0.01 to 1.0 parts by weight based on a total of 100 parts by weight of the composition. For example, it may be included in an amount of 0.01 to 0.9 parts by weight, 0.01 to 0.8 parts by weight, 0.01 to 0.7 parts by weight, 0.01 to 0.6 parts by weight, 0.01 to 0.5 parts by weight, 0.01 to 0.4 parts by weight, 0.01 to 0.3 parts by weight, 0.01 to 0.2 parts by weight, 0.01 to 0.1 parts by weight, 0.01 to 0.05 parts by weight, 0.02 to 1.0 parts by weight, 0.02 to 0.9 parts by weight, 0.02 to 0.8 parts by weight, 0.02 to 0.7 parts by weight, 0.02 to 0.6 parts by weight, 0.02 to 0.5 parts by weight, 0.02 to 0.4 parts by weight, 0.02 to 0.3 parts by weight, 0.02 to 0.2 parts by weight, 0.02 to 0.1 parts by weight, 0.02 to 0.05 parts by weight, 0.03 to 1.0 parts by weight, 0.03 to 0.9 parts by weight, 0.03 to 0.8 parts by weight, 0.03 to 0.7 parts by weight, 0.03 to 0.6 parts by weight, 0.03 to 0.5 parts by weight, 0.03 to 0.4 parts by weight, 0.03 to 0.3 parts by weight, 0.03 to 0.2 parts by weight, 0.03 to 0.1 parts by weight, 0.03 to 0.05 parts by weight, 0.04 to 1.0 parts by weight, 0.04 to 0.9 parts by weight, 0.04 to 0.8 parts by weight, 0.04 to 0.7 parts by weight, 0.04 to 0.6 parts by weight, 0.04 to 0.5 parts by weight, 0.04 to 0.4 parts by weight, 0.04 to 0.3 parts by weight, 0.04 to 0.2 parts by weight, 0.04 to 0.1 parts by weight, 0.04 to 0.05 parts by weight, 0.05 to 1.0 parts by weight, 0.05 to 0.9 parts by weight, 0.05 to 0.8 parts by weight, 0.05 to 0.7 parts by weight, 0.05 to 0.6 parts by weight, 0.05 to 0.5 parts by weight, 0.05 to 0.4 parts by weight, 0.05 to 0.3 parts by weight, 0.05 to 0.2 parts by weight, 0.05 to 0.1 parts by weight, 0.06 to 1.0 parts by weight, 0.06 to 0.9 parts by weight, 0.06 to 0.8 parts by weight, 0.06 to 0.7 parts by weight, 0.06 to 0.6 parts by weight, 0.06 to 0.5 parts by weight, 0.06 to 0.4 parts by weight, 0.06 to 0.3 parts by weight, 0.06 to 0.2 parts by weight, 0.06 to 0.1 parts by weight, 0.07 to 1.0 parts by weight, 0.07 to 0.9 parts by weight, 0.07 to 0.8 parts by weight, 0.07 to 0.7 parts by weight, 0.07 to 0.6 parts by weight, 0.07 to 0.5 parts by weight, 0.07 to 0.4 parts by weight, 0.07 to 0.3 parts by weight, 0.07 to 0.2 parts by weight, 0.07 to 0.1 parts by weight, 0.08 to 1.0 parts by weight, 0.08 to 0.9 parts by weight, 0.08 to 0.8 parts by weight, 0.08 to 0.7 parts by weight, 0.08 to 0.6 parts by weight, 0.08 to 0.5 parts by weight, 0.08 to 0.4 parts by weight, 0.08 to 0.3 parts by weight, 0.08 to 0.2 parts by weight, 0.08 to 0.1 parts by weight, 0.09 to 1.0 parts by weight, 0.09 to 0.9 parts by weight, 0.09 to 0.8 parts by weight, 0.09 to 0.7 parts by weight, 0.09 to 0.6 parts by weight, 0.09 to 0.5 parts by weight, 0.09 to 0.4 parts by weight, 0.09 to 0.3 parts by weight, 0.09 to 0.2 parts by weight, or 0.09 to 0.1 parts by weight, but is not limited thereto. For example, the Tween 20 may be included in an amount of 0.5 parts by weight based on a total of 100 parts by weight of the composition.

When the Tween 20 is included in an amount of 1.0 parts by weight or more, it induces foaming during the process and has adverse effects on the membrane used in the concentration process, thereby leading to the problem of reduced concentration yield (efficiency). Additionally, as the amount of additive increases, there is the problem of increased production cost, and in feed applications, it may cause a chemical odor.

Specifically, the sorbitol or glycerol may each be included in an amount of 0.1 to 10.0 parts by weight based on a total of 100 parts by weight of the composition. For example, it may be included in an amount of 0.1 to 9.0 parts by weight, 0.1 to 8.0 parts by weight, 0.1 to 7.0 parts by weight, 0.1 to 6.0 parts by weight, 0.1 to 5.0 parts by weight, 0.1 to 4.0 parts by weight, 0.1 to 3.0 parts by weight, 0.1 to 2.0 parts by weight, 0.1 to 1.0 parts by weight, 0.1 to 0.5 parts by weight, 0.2 to 10.0 parts by weight, 0.2 to 9.0 parts by weight, 0.2 to 8.0 parts by weight, 0.2 to 7.0 parts by weight, 0.2 to 6.0 parts by weight, 0.2 to 5.0 parts by weight, 0.2 to 4.0 parts by weight, 0.2 to 3.0 parts by weight, 0.2 to 2.0 parts by weight, 0.2 to 1.0 parts by weight, 0.2 to 0.5 parts by weight, 0.3 to 10.0 parts by weight, 0.3 to 9.0 parts by weight, 0.3 to 8.0 parts by weight, 0.3 to 7.0 parts by weight, 0.3 to 6.0 parts by weight, 0.3 to 5.0 parts by weight, 0.3 to 4.0 parts by weight, 0.3 to 3.0 parts by weight, 0.3 to 2.0 parts by weight, 0.3 to 1.0 parts by weight, 0.3 to 0.5 parts by weight, 0.4 to 10.0 parts by weight, 0.4 to 9.0 parts by weight, 0.4 to 8.0 parts by weight, 0.4 to 7.0 parts by weight, 0.4 to 6.0 parts by weight, 0.4 to 5.0 parts by weight, 0.4 to 4.0 parts by weight, 0.4 to 3.0 parts by weight, 0.4 to 2.0 parts by weight, 0.4 to 1.0 parts by weight, 0.4 to 0.5 parts by weight, 0.5 to 10.0 parts by weight, 0.5 to 9.0 parts by weight, 0.5 to 8.0 parts by weight, 0.5 to 7.0 parts by weight, 0.5 to 6.0 parts by weight, 0.5 to 5.0 parts by weight, 0.5 to 4.0 parts by weight, 0.5 to 3.0 parts by weight, 0.5 to 2.0 parts by weight, 0.5 to 1.0 parts by weight, 0.6 to 10.0 parts by weight, 0.6 to 9.0 parts by weight, 0.6 to 8.0 parts by weight, 0.6 to 7.0 parts by weight, 0.6 to 6.0 parts by weight, 0.6 to 5.0 parts by weight, 0.6 to 4.0 parts by weight, 0.6 to 3.0 parts by weight, 0.6 to 2.0 parts by weight, 0.6 to 1.0 parts by weight, 0.7 to 10.0 parts by weight, 0.7 to 9.0 parts by weight, 0.7 to 8.0 parts by weight, 0.7 to 7.0 parts by weight, 0.7 to 6.0 parts by weight, 0.7 to 5.0 parts by weight, 0.7 to 4.0 parts by weight, 0.7 to 3.0 parts by weight, 0.7 to 2.0 parts by weight, 0.7 to 1.0 parts by weight, 0.8 to 10.0 parts by weight, 0.8 to 9.0 parts by weight, 0.8 to 8.0 parts by weight, 0.8 to 7.0 parts by weight, 0.8 to 6.0 parts by weight, 0.8 to 5.0 parts by weight, 0.8 to 4.0 parts by weight, 0.8 to 3.0 parts by weight, 0.8 to 2.0 parts by weight, 0.8 to 1.0 parts by weight, 0.9 to 10.0 parts by weight, 0.9 to 9.0 parts by weight, 0.9 to 8.0 parts by weight, 0.9 to 7.0 parts by weight, 0.9 to 6.0 parts by weight, 0.9 to 5.0 parts by weight, 0.9 to 4.0 parts by weight, 0.9 to 3.0 parts by weight, 0.9 to 2.0 parts by weight, 0.9 to 1.0 parts by weight, 1.0 to 10.0 parts by weight, 1.0 to 9.0 parts by weight, 1.0 to 8.0 parts by weight, 1.0 to 7.0 parts by weight, 1.0 to 6.0 parts by weight, 1.0 to 5.0 parts by weight, 1.0 to 4.0 parts by weight, 1.0 to 3.0 parts by weight, 1.0 to 2.0 parts by weight, 1.5 to 10.0 parts by weight, 1.5 to 9.0 parts by weight, 1.5 to 8.0 parts by weight, 1.5 to 7.0 parts by weight, 1.5 to 6.0 parts by weight, 1.5 to 5.0 parts by weight, 1.5 to 4.0 parts by weight, 1.5 to 3.0 parts by weight, 1.5 to 2.0 parts by weight, 2.0 to 10.0 parts by weight, 2.0 to 9.0 parts by weight, 2.0 to 8.0 parts by weight, 2.0 to 7.0 parts by weight, 2.0 to 6.0 parts by weight, 2.0 to 5.0 parts by weight, 2.0 to 4.0 parts by weight, 2.0 to 3.0 parts by weight, 3.0 to 10.0 parts by weight, 3.0 to 9.0 parts by weight, 3.0 to 8.0 parts by weight, 3.0 to 7.0 parts by weight, 3.0 to 6.0 parts by weight, 3.0 to 5.0 parts by weight, 3.0 to 4.0 parts by weight, 4.0 to 10.0 parts by weight, 4.0 to 9.0 parts by weight, 4.0 to 8.0 parts by weight, 4.0 to 7.0 parts by weight, 4.0 to 6.0 parts by weight, 4.0 to 5.0 parts by weight, 5.0 to 10.0 parts by weight, 5.0 to 9.0 parts by weight, 5.0 to 8.0 parts by weight, 5.0 to 7.0 parts by weight, 5.0 to 6.0 parts by weight, 6.0 to 10.0 parts by weight, 6.0 to 9.0 parts by weight, 6.0 to 8.0 parts by weight, 6.0 to 7.0 parts by weight, 7.0 to 10.0 parts by weight, 7.0 to 9.0 parts by weight, 7.0 to 8.0 parts by weight, 8.0 to 10.0 parts by weight, 8.0 to 9.0 parts by weight, or 9.0 to 10.0 parts by weight, but is not limited thereto. For example, the sorbitol or glycerol may each be included in an amount of 1.0 parts by weight based on a total of 100 parts by weight of the composition.

When the sorbitol or glycerol is included in an amount of 10.0 parts by weight or more, the viscosity of the liquid increases, thereby causing the problem of hindering the flux in the concentration process, and when added in an excessive amount, the concentration process may fail to proceed. Additionally, as the added amount increases, there is the problem of increased production cost.

By comprising the Tween 20, and sorbitol or glycerol, in the above amount ranges, the stability of phospholipase is increased. Thus, it solves the problem of enzyme inactivation during purification and concentration steps in the production of phospholipase, thereby exhibiting a superior effect in increasing product yield and economic feasibility.

Tween 20, sorbitol, and glycerol used in the present disclosure are not particularly limited, and any Tween 20, sorbitol, and glycerol commonly used in the relevant or similar technical field may be used.

The composition for stabilizing phospholipase of the present disclosure may have improved stability of phospholipase compared to a composition that does not comprise: (i) Tween 20; and (ii) sorbitol, glycerol, or a combination thereof.

Another aspect of the present disclosure provides a method for stabilizing phospholipase, comprising: adding, to a composition comprising phospholipase, a composition for stabilizing phospholipase comprising: (i) Tween 20, and (ii) sorbitol, glycerol, or a combination thereof, to prepare a mixed composition; and concentrating the mixed composition.

The composition for stabilizing phospholipase is as described above.

In one embodiment, the composition for stabilizing phospholipase of the present disclosure may comprise: Tween 20 and sorbitol; Tween 20 and glycerol; or Tween 20, sorbitol, and glycerol.

Specifically, the Tween 20 may be included in an amount of 0.01 to 1.0 parts by weight based on a total of 100 parts by weight of the composition. For example, it may be included in an amount of 0.01 to 0.9 parts by weight, 0.01 to 0.8 parts by weight, 0.01 to 0.7 parts by weight, 0.01 to 0.6 parts by weight, 0.01 to 0.5 parts by weight, 0.01 to 0.4 parts by weight, 0.01 to 0.3 parts by weight, 0.01 to 0.2 parts by weight, 0.01 to 0.1 parts by weight, 0.01 to 0.05 parts by weight, 0.02 to 1.0 parts by weight, 0.02 to 0.9 parts by weight, 0.02 to 0.8 parts by weight, 0.02 to 0.7 parts by weight, 0.02 to 0.6 parts by weight, 0.02 to 0.5 parts by weight, 0.02 to 0.4 parts by weight, 0.02 to 0.3 parts by weight, 0.02 to 0.2 parts by weight, 0.02 to 0.1 parts by weight, 0.02 to 0.05 parts by weight, 0.03 to 1.0 parts by weight, 0.03 to 0.9 parts by weight, 0.03 to 0.8 parts by weight, 0.03 to 0.7 parts by weight, 0.03 to 0.6 parts by weight, 0.03 to 0.5 parts by weight, 0.03 to 0.4 parts by weight, 0.03 to 0.3 parts by weight, 0.03 to 0.2 parts by weight, 0.03 to 0.1 parts by weight, 0.03 to 0.05 parts by weight, 0.04 to 1.0 parts by weight, 0.04 to 0.9 parts by weight, 0.04 to 0.8 parts by weight, 0.04 to 0.7 parts by weight, 0.04 to 0.6 parts by weight, 0.04 to 0.5 parts by weight, 0.04 to 0.4 parts by weight, 0.04 to 0.3 parts by weight, 0.04 to 0.2 parts by weight, 0.04 to 0.1 parts by weight, 0.04 to 0.05 parts by weight, 0.05 to 1.0 parts by weight, 0.05 to 0.9 parts by weight, 0.05 to 0.8 parts by weight, 0.05 to 0.7 parts by weight, 0.05 to 0.6 parts by weight, 0.05 to 0.5 parts by weight, 0.05 to 0.4 parts by weight, 0.05 to 0.3 parts by weight, 0.05 to 0.2 parts by weight, 0.05 to 0.1 parts by weight, 0.06 to 1.0 parts by weight, 0.06 to 0.9 parts by weight, 0.06 to 0.8 parts by weight, 0.06 to 0.7 parts by weight, 0.06 to 0.6 parts by weight, 0.06 to 0.5 parts by weight, 0.06 to 0.4 parts by weight, 0.06 to 0.3 parts by weight, 0.06 to 0.2 parts by weight, 0.06 to 0.1 parts by weight, 0.07 to 1.0 parts by weight, 0.07 to 0.9 parts by weight, 0.07 to 0.8 parts by weight, 0.07 to 0.7 parts by weight, 0.07 to 0.6 parts by weight, 0.07 to 0.5 parts by weight, 0.07 to 0.4 parts by weight, 0.07 to 0.3 parts by weight, 0.07 to 0.2 parts by weight, 0.07 to 0.1 parts by weight, 0.08 to 1.0 parts by weight, 0.08 to 0.9 parts by weight, 0.08 to 0.8 parts by weight, 0.08 to 0.7 parts by weight, 0.08 to 0.6 parts by weight, 0.08 to 0.5 parts by weight, 0.08 to 0.4 parts by weight, 0.08 to 0.3 parts by weight, 0.08 to 0.2 parts by weight, 0.08 to 0.1 parts by weight, 0.09 to 1.0 parts by weight, 0.09 to 0.9 parts by weight, 0.09 to 0.8 parts by weight, 0.09 to 0.7 parts by weight, 0.09 to 0.6 parts by weight, 0.09 to 0.5 parts by weight, 0.09 to 0.4 parts by weight, 0.09 to 0.3 parts by weight, 0.09 to 0.2 parts by weight, or 0.09 to 0.1 parts by weight, but is not limited thereto.

Specifically, the sorbitol or glycerol may each be included in an amount of 0.1 to 10.0 parts by weight based on a total of 100 parts by weight of the composition. For example, it may be included in an amount of 0.1 to 9.0 parts by weight, 0.1 to 8.0 parts by weight, 0.1 to 7.0 parts by weight, 0.1 to 6.0 parts by weight, 0.1 to 5.0 parts by weight, 0.1 to 4.0 parts by weight, 0.1 to 3.0 parts by weight, 0.1 to 2.0 parts by weight, 0.1 to 1.0 parts by weight, 0.1 to 0.5 parts by weight, 0.2 to 10.0 parts by weight, 0.2 to 9.0 parts by weight, 0.2 to 8.0 parts by weight, 0.2 to 7.0 parts by weight, 0.2 to 6.0 parts by weight, 0.2 to 5.0 parts by weight, 0.2 to 4.0 parts by weight, 0.2 to 3.0 parts by weight, 0.2 to 2.0 parts by weight, 0.2 to 1.0 parts by weight, 0.2 to 0.5 parts by weight, 0.3 to 10.0 parts by weight, 0.3 to 9.0 parts by weight, 0.3 to 8.0 parts by weight, 0.3 to 7.0 parts by weight, 0.3 to 6.0 parts by weight, 0.3 to 5.0 parts by weight, 0.3 to 4.0 parts by weight, 0.3 to 3.0 parts by weight, 0.3 to 2.0 parts by weight, 0.3 to 1.0 parts by weight, 0.3 to 0.5 parts by weight, 0.4 to 10.0 parts by weight, 0.4 to 9.0 parts by weight, 0.4 to 8.0 parts by weight, 0.4 to 7.0 parts by weight, 0.4 to 6.0 parts by weight, 0.4 to 5.0 parts by weight, 0.4 to 4.0 parts by weight, 0.4 to 3.0 parts by weight, 0.4 to 2.0 parts by weight, 0.4 to 1.0 parts by weight, 0.4 to 0.5 parts by weight, 0.5 to 10.0 parts by weight, 0.5 to 9.0 parts by weight, 0.5 to 8.0 parts by weight, 0.5 to 7.0 parts by weight, 0.5 to 6.0 parts by weight, 0.5 to 5.0 parts by weight, 0.5 to 4.0 parts by weight, 0.5 to 3.0 parts by weight, 0.5 to 2.0 parts by weight, 0.5 to 1.0 parts by weight, 0.6 to 10.0 parts by weight, 0.6 to 9.0 parts by weight, 0.6 to 8.0 parts by weight, 0.6 to 7.0 parts by weight, 0.6 to 6.0 parts by weight, 0.6 to 5.0 parts by weight, 0.6 to 4.0 parts by weight, 0.6 to 3.0 parts by weight, 0.6 to 2.0 parts by weight, 0.6 to 1.0 parts by weight, 0.7 to 10.0 parts by weight, 0.7 to 9.0 parts by weight, 0.7 to 8.0 parts by weight, 0.7 to 7.0 parts by weight, 0.7 to 6.0 parts by weight, 0.7 to 5.0 parts by weight, 0.7 to 4.0 parts by weight, 0.7 to 3.0 parts by weight, 0.7 to 2.0 parts by weight, 0.7 to 1.0 parts by weight, 0.8 to 10.0 parts by weight, 0.8 to 9.0 parts by weight, 0.8 to 8.0 parts by weight, 0.8 to 7.0 parts by weight, 0.8 to 6.0 parts by weight, 0.8 to 5.0 parts by weight, 0.8 to 4.0 parts by weight, 0.8 to 3.0 parts by weight, 0.8 to 2.0 parts by weight, 0.8 to 1.0 parts by weight, 0.9 to 10.0 parts by weight, 0.9 to 9.0 parts by weight, 0.9 to 8.0 parts by weight, 0.9 to 7.0 parts by weight, 0.9 to 6.0 parts by weight, 0.9 to 5.0 parts by weight, 0.9 to 4.0 parts by weight, 0.9 to 3.0 parts by weight, 0.9 to 2.0 parts by weight, 0.9 to 1.0 parts by weight, 1.0 to 10.0 parts by weight, 1.0 to 9.0 parts by weight, 1.0 to 8.0 parts by weight, 1.0 to 7.0 parts by weight, 1.0 to 6.0 parts by weight, 1.0 to 5.0 parts by weight, 1.0 to 4.0 parts by weight, 1.0 to 3.0 parts by weight, 1.0 to 2.0 parts by weight, 1.5 to 10.0 parts by weight, 1.5 to 9.0 parts by weight, 1.5 to 8.0 parts by weight, 1.5 to 7.0 parts by weight, 1.5 to 6.0 parts by weight, 1.5 to 5.0 parts by weight, 1.5 to 4.0 parts by weight, 1.5 to 3.0 parts by weight, 1.5 to 2.0 parts by weight, 2.0 to 10.0 parts by weight, 2.0 to 9.0 parts by weight, 2.0 to 8.0 parts by weight, 2.0 to 7.0 parts by weight, 2.0 to 6.0 parts by weight, 2.0 to 5.0 parts by weight, 2.0 to 4.0 parts by weight, 2.0 to 3.0 parts by weight, 3.0 to 10.0 parts by weight, 3.0 to 9.0 parts by weight, 3.0 to 8.0 parts by weight, 3.0 to 7.0 parts by weight, 3.0 to 6.0 parts by weight, 3.0 to 5.0 parts by weight, 3.0 to 4.0 parts by weight, 4.0 to 10.0 parts by weight, 4.0 to 9.0 parts by weight, 4.0 to 8.0 parts by weight, 4.0 to 7.0 parts by weight, 4.0 to 6.0 parts by weight, 4.0 to 5.0 parts by weight, 5.0 to 10.0 parts by weight, 5.0 to 9.0 parts by weight, 5.0 to 8.0 parts by weight, 5.0 to 7.0 parts by weight, 5.0 to 6.0 parts by weight, 6.0 to 10.0 parts by weight, 6.0 to 9.0 parts by weight, 6.0 to 8.0 parts by weight, 6.0 to 7.0 parts by weight, 7.0 to 10.0 parts by weight, 7.0 to 9.0 parts by weight, 7.0 to 8.0 parts by weight, 8.0 to 10.0 parts by weight, 8.0 to 9.0 parts by weight, or 9.0 to 10.0 parts by weight, but is not limited thereto.

In one embodiment, the concentrating step may be performed by a method selected from the group consisting of ultrafiltration, column chromatography, solid-phase extraction, reverse phase high-performance liquid chromatography, and a combination thereof, but is not limited thereto.

Specifically, the concentrating step may be performed by an ultrafiltration method, but is not limited thereto.

Inactivation of enzymes often occurs during the enzyme production process due to denaturation of the protein during purification and concentration steps, thereby leading to the problem of enzyme inactivation, *i.e.,* loss of enzyme activity. For example, during purification and concentration of the enzyme by ultrafiltration, protein denaturation (aggregation or denaturation) occurs due to shear stress and the like, resulting in enzyme inactivation.

In the method for stabilizing phospholipase of the present disclosure, the composition for stabilizing phospholipase may increase the stability of the phospholipase after the concentrating step.

Specifically, the phospholipase activity in the mixed composition after the concentration step is retained at a range of 80% or more and less than 100%, specifically 85% or more and 99% or less, and more specifically 89% or more and 99% or less, of the phospholipase activity of the mixed composition before the concentrating step.

In one embodiment, the phospholipase may be a phospholipase derived from a *Trichoderma* sp. microorganism.

In one embodiment, the composition comprising phospholipase may be obtained by culturing a *Trichoderma* sp. microorganism in a medium.

In one embodiment of the foregoing embodiments, the composition comprising phospholipase may comprise a *Trichoderma* sp. microorganism, a culture of the microorganism, a fermentation product of the microorganism, or a combination of two or more thereof.

As used herein, the term "culturing" refers to growing the strain of the present disclosure under properly controlled environmental conditions. The culturing process of the present disclosure may be performed in a suitable medium known in the art under suitable culturing conditions known in the art. Such a culturing process may be readily adjusted and used by those skilled in the art according to the selected strain. Specifically, the culturing may be batch culturing, continuous culturing, and fed-batch culturing, but is not limited thereto.

As used herein, the term "medium" refers to a mixed substance containing nutrients required to culture the microorganism of the present disclosure as a main component, and the medium supplies nutrients, growth factors, *etc.,* including water, which are indispensable for survival and development. Specifically, any medium and culture conditions may be used for culturing the strain of the present disclosure without particular limitation, as long as the medium is used for the common culture of microorganisms. The microorganism of the present disclosure may be cultured in a common medium containing suitable carbon sources, nitrogen sources, phosphorus sources, inorganic compounds, amino acids, and/or vitamins, *etc.,* while controlling the temperature, pH, *etc.* under aerobic conditions.

In the present disclosure, the carbon sources include carbohydrates such as glucose, saccharose, lactose, fructose, sucrose, and maltose; sugar alcohols such as mannitol and sorbitol; organic acids such as pyruvic acid, lactic acid, and citric acid; or amino acids such as glutamic acid, methionine, and lysine. Additionally, natural organic nutrients such as starch hydrolysates, molasses, blackstrap molasses, rice bran, cassava, sugarcane bagasse, and corn steep liquor may be used. Specifically, carbohydrates such as glucose and sterilized pretreated molasses (*i.e.,* molasses converted into reducing sugars) may be used, and other various carbon sources in appropriate amounts may be used without limitation. These carbon sources may be used alone or in combination of two or more, but are not limited thereto.

As the nitrogen sources, inorganic nitrogen sources such as ammonia, ammonium sulfate, ammonium chloride, ammonium acetate, ammonium phosphate, ammonium carbonate, and ammonium nitrate, or organic nitrogen sources such as amino acids such as glutamic acid, methionine, and glutamine, peptone, NZ-amine, meat extract, yeast extract, malt extract, corn steep liquor, casein hydrolysate, fish or decomposition products thereof, and skim soybean cake or decomposition products thereof may be used. These nitrogen sources may be used alone or in combination of two or more, but are not limited thereto.

As the phosphorus sources, monobasic potassium phosphate, dibasic potassium phosphate, or the corresponding sodium-containing salts may be used. As the inorganic compounds, sodium chloride, calcium chloride, iron chloride, magnesium sulfate, iron sulfate, manganese sulfate, calcium carbonate, *etc*. may be used. In addition, amino acids, vitamins, and/or suitable precursors, *etc.* may be included. These components or precursors may be added to the medium in a batchwise or continuous manner. However, the medium is not limited thereto.

During the culturing of the microorganism of the present disclosure, compounds such as ammonium hydroxide, potassium hydroxide, ammonia, phosphoric acid, and sulfuric acid may be added to the medium in an appropriate manner to adjust the pH of the medium. During the culturing, an antifoaming agent such as fatty acid polyglycol ester may be used to suppress foaming. To maintain an aerobic state of the medium, oxygen or oxygen-containing gas may be injected into the medium. To maintain an anaerobic or microaerobic state of the medium, no gas or nitrogen, hydrogen, or carbon dioxide gas may be injected. However, the culturing conditions are not limited thereto.

In the culturing of the present disclosure, the culturing temperature may be maintained at 27°C to 37°C, specifically 30°C to 33°C, and the culturing may be performed for about 20 to 120 hours, but the culturing conditions are not limited thereto.

As used herein, the term "culture" refers to a culture solution, a concentrated culture solution, a dried product of the culture solution, a culture filtrate, a concentrated culture filtrate, or a dried product of the culture filtrate obtained by culturing a specific microorganism in a culture medium. The culture solution means a solution containing a specific microorganism, whereas the term "culture filtrate" means a solution that substantially does not contain the specific microorganism (wherein "substantially" means that the specific microorganism separated by filtration or the like is excluded, but does not mean that the microorganism is completely absent from the filtrate). The culture is not limited in its form, and, for example, may be in the form of a liquid, an emulsion, or a solid. Specifically, according to the purposes of the present disclosure, the culture may comprise phospholipase.

As used herein, the term "fermentation" refers to a process in which a microorganism decomposes organic matter using enzymes possessed by the microorganism, excluding putrefactive reactions. Fermentation reactions and putrefactive reactions proceed using similar processes. However, upon decomposition, fermentation produces useful substances, whereas putrefaction produces bad odors or harmful substances.

In the present disclosure, the method for obtaining a fermentation product from the strain is not particularly limited and can be obtained according to methods conventionally used in the relevant or similar technical field.

In the present disclosure, the term "fermentation product" includes not only the fermented substance itself but may also include any type of material comprising the fermentation product derived from the strain, including: a culture medium of the strain in which the strain and the culture coexist; a fermentation product obtained by filtering the strain from the culture medium; a fermentation product obtained by sterilizing and filtering the strain in the culture medium; an extract obtained by extracting the fermentation product or the culture medium comprising the same; a diluted solution or a concentrated solution obtained by diluting or concentrating the fermentation product or an extract thereof; a dried product obtained by drying the fermentation product or an extract thereof; and a lysate obtained by collecting and disrupting the cell body of the strain.

In the method of the present disclosure, culturing of microorganisms may be performed using any culturing conditions and culturing methods known in the art. Such a culturing process may be readily adjusted and used by those skilled in the art according to the selected strain.

Still another aspect of the present disclosure provides the use of a composition comprising (i) Tween 20 and (ii) sorbitol, glycerol, or a combination thereof for stabilizing phospholipase.

### [Mode for Carrying Out the Invention]

Hereinafter, the present disclosure will be described in more detail by way of exemplary embodiments. However, the following Examples are given for illustrative purposes only, and the scope of the invention is not intended to be limited by these Examples. Meanwhile, the technical descriptions absent in the present disclosure may be sufficiently understood and readily practiced by those of ordinary skill in the art of the present disclosure or related art.

### Example. Evaluation of the Effect of the Composition for Stabilizing Phospholipase in Preventing Enzyme Inactivation

A composition (fermentation broth) comprising phospholipase was prepared using a fed-batch culturing process. Based on the dissolved oxygen (DO) level as an indicator, feed medium was supplied at the point where DO began to rise. The components of the present fermentation medium were lactose, corn steep liquor, calcium chloride, disodium phosphate, ammonium sulfate, and magnesium sulfate. The feed medium was composed of lactose, manganese sulfate, ferrous sulfate, and zinc sulfate. Meanwhile, regarding the fermentation conditions, the culture temperature was maintained at 32 °C and the stirring speed was adjusted such that DO limitation does not occur.

For Tween 20 (Sinopharm Chemical Reagent Co., Ltd.), sorbitol (Shandong Tianli Pharmaceutical Co., Ltd.), and glycerol (Zibo Yingzhao Chemical Technology Co., Ltd.), commercially available products were used.

Based on the weight of the fermentation broth, Tween 20 at 0.05%(w/w), sorbitol at 1%(w/w), glycerol at 1%(w/w), a combination of Tween 20 at 0.05%(w/w) and sorbitol at 1%(w/w), or a combination of Tween 20 at 0.05%(w/w) and glycerol at 1%(w/w) was respectively added to fermentation broths comprising the produced phospholipase, while a fermentation broth comprising phospholipase without any added stabilizer was used as a control.

Subsequently, an ultrafiltration concentration process (UF process) was performed using a lab-scale TFF system (Millipore) equipped with a 20 kDa membrane (Millipore, Pellicon XL). Specifically, concentration was carried out at a volumetric concentration factor (VCF) of 2.5.

Thereafter, the unit process yield was evaluated by measuring enzyme activity in the fermentation broth before concentration, and in the concentrate, permeate, and wash water after concentration. Specifically, one unit (U) of phospholipase enzyme activity was measured as the amount of enzyme required to release 1 µmol of unsaturated polygalacturonic acid per minute from a 6 mg/mL polygalacturonic acid solution at 50°C and pH 7.73.

The results are shown in Tables 1 to 6 below. Specifically, Table 1 shows the results of the control group; Table 2, the group with 0.05% (w/w) Tween 20; Table 3, the group with 1% (w/w) sorbitol; Table 4, the group with 1% (w/w) glycerol; Table 5, the group with the combination of 0.05% (w/w) Tween 20 and 1% (w/w) sorbitol; and Table 6, the group with the combination of 0.05% (w/w) Tween 20 and 1% (w/w) glycerol.

**[Table 1]**

| Sample | Volume | Activity | Tot. Activity | Yield |
|---|---|---|---|---|
| | mL | Unit/mL | Unit | % |
| Feed | 500.0 | 45.3 | 22650.0 | 100.0 |
| Concentrate | 201.0 | 49.8 | 10009.8 | 44.2 |
| Permeate | 296.0 | 0.2 | 59.2 | 0.3 |
| System wash water | 42.5 | 4.5 | 191.3 | 0.8 |

**[Table 2]**

| Sample | Volume | Activity | Tot. Activity | Yield |
|---|---|---|---|---|
| | mL | Unit/mL | Unit | % |
| Feed | 500.0 | 45.3 | 22650.0 | 100.0 |
| Concentrate | 200.4 | 101.2 | 20280.5 | 89.5 |
| Permeate | 297.0 | 0.4 | 118.8 | 0.5 |
| System wash water | 41.0 | 2.2 | 90.2 | 0.4 |

**[Table 3]**

| Sample | Volume | Activity | Tot. Activity | Yield |
|---|---|---|---|---|
| | mL | Unit/mL | Unit | % |
| Feed | 500.0 | 45.3 | 22650.0 | 100.0 |
| Concentrate | 199.8 | 100.3 | 20039.9 | 88.5 |
| Permeate | 297.0 | 0.4 | 118.8 | 0.5 |
| System wash water | 40.8 | 3.7 | 149.7 | 0.7 |

**[Table 4]**

| Sample | Volume | Activity | Tot. Activity | Yield |
|---|---|---|---|---|
| | mL | Unit/mL | Unit | % |
| Feed | 500.0 | 45.3 | 22650.0 | 100.0 |
| Concentrate | 200.5 | 98.0 | 19645.1 | 86.7 |
| Permeate | 298.5 | 0.3 | 89.6 | 0.4 |
| System wash water | 41.2 | 3.3 | 136.0 | 0.6 |

**[Table 5]**

| Sample | Volume | Activity | Tot. Activity | Yield |
|---|---|---|---|---|
| | mL | Unit/mL | Unit | % |
| Feed | 500.0 | 45.3 | 22650.0 | 100.0 |
| Concentrate | 199.0 | 112.6 | 22407.4 | 98.9 |
| Permeate | 298.8 | 0.1 | 29.9 | 0.1 |
| System wash water | 40.9 | 2.4 | 98.2 | 0.4 |

**[Table 6]**

| Sample | Volume | Activity | Tot. Activity | Yield |
|---|---|---|---|---|
| | mL | Unit/mL | Unit | % |
| Feed | 500.0 | 45.3 | 22650.0 | 100.0 |
| Concentrate | 199.4 | 110.4 | 22013.8 | 97.2 |
| Permeate | 297.5 | 0.1 | 29.8 | 0.1 |
| System wash water | 41.5 | 2.8 | 116.2 | 0.5 |

As a result, as shown in Table 1, the case of the control group without any added stabilizer had a UF concentrate yield of 44.2%, confirming that enzyme inactivation occurred. In addition, since 1.1% of activity was detected in the UF permeate and system wash water, it was confirmed that an unknown loss of 54.7% occurred during the process. This suggests that structural instability of the enzyme occurred during the UF process, resulting in enzyme inactivation.

Furthermore, as shown in Tables 2 to 4, when Tween 20, sorbitol, or glycerol was added individually as a stabilizer, the UF concentrate yields were 89.5%, 88.5%, and 86.7%, respectively. Therefore, compared to the control, the yields were improved by 45.3%p, 44.3%p, and 42.5%p, respectively. However, when Tween 20, sorbitol, or glycerol was added individually as a stabilizer, unknown losses of 9.5%, 10.3%, and 12.3%, respectively, were observed, indicating that enzyme inactivation still occurred.

In contrast, as shown in Tables 5 and 6, the combinations of Tween 20 at 0.05%(w/w) + sorbitol at 1%(w/w) and Tween 20 at 0.05%(w/w) + glycerol at 1%(w/w) exhibited an effect in preventing enzyme inactivation. When the combinations of Tween 20 + sorbitol and Tween 20 + glycerol were added to the composition comprising phospholipase, the UF concentrate yields were 98.9% and 97.2%, respectively, which confirms improvements of 54.7%p and 53.0%p over the control. In particular, the unknown losses were reduced to 0.5% and 2.2%, corresponding to improvements of 54.2%p and 52.5%p, respectively, compared to the control. Compared to the cases where Tween 20, sorbitol, or glycerol was added individually, an improvement of up to 11.8%p was confirmed.

These results suggest that the specific combination of Tween 20 and sorbitol or glycerol according to the present disclosure can prevent inactivation of phospholipase more effectively than when each component is used alone, and thus can serve as a composition specifically for stabilizing phospholipase.

Through the above, it was confirmed that the composition for stabilizing phospholipase of the present disclosure increases the stability of phospholipase by comprising Tween 20 and sorbitol or glycerol, thereby solving the problem of enzyme inactivation during purification and concentration steps in the production of phospholipase and thus exhibiting a superior effect in increasing product yield and economic feasibility.

As set forth above, those skilled in the art will be able to understand that the present disclosure may be embodied in other specific forms without departing from the technical spirit or essential characteristics thereof. In this regard, it should be understood that the foregoing examples are illustrative in all respects and are not to be construed as limiting. The scope of the present disclosure should be construed as including the meaning and scope of the appended claims rather than the detailed description, and all changes or variations derived from the equivalent concepts fall within the scope of the present disclosure.

## Claims

1. A composition for stabilizing phospholipase, comprising:
(i) Tween 20; and
(ii) sorbitol, glycerol, or a combination thereof.

2. The composition for stabilizing phospholipase according to claim 1, wherein the composition comprises Tween 20 and sorbitol.

3. The composition for stabilizing phospholipase according to claim 1, wherein the composition comprises Tween 20 and glycerol.

4. The composition for stabilizing phospholipase according to claim 1, wherein the Tween 20 is comprised in an amount of 0.01 to 1.0 parts by weight based on a total of 100 parts by weight of the composition.

5. The composition for stabilizing phospholipase according to claim 1, wherein the Tween 20 is comprised in an amount of 0.01 to 1.0 parts by weight based on a total of 100 parts by weight of the composition, and
wherein the sorbitol or glycerol is comprised in an amount of 0.1 to 10.0 parts by weight based on a total of 100 parts by weight of the composition.

6. The composition for stabilizing phospholipase according to claim 1, wherein the composition has improved stability of phospholipase compared to a composition that does not comprise: (i) Tween 20; and (ii) sorbitol, glycerol, or a combination thereof.

7. The composition for stabilizing phospholipase according to claim 1, wherein the phospholipase is derived from a *Trichoderma* sp. microorganism.

8. A method for stabilizing phospholipase, comprising:
adding, to a composition comprising phospholipase, a composition for stabilizing phospholipase comprising: (i) Tween 20, and (ii) sorbitol, glycerol, or a combination thereof, to prepare a mixed composition; and
concentrating the mixed composition.

9. The method for stabilizing phospholipase according to claim 8, wherein the composition for stabilizing phospholipase comprises Tween 20 and sorbitol.

10. The method for stabilizing phospholipase according to claim 8, wherein the composition for stabilizing phospholipase comprises Tween 20 and glycerol.

11. The method for stabilizing phospholipase according to claim 8, wherein the concentrating step is performed by a method selected from the group consisting of ultrafiltration, column chromatography, solid-phase extraction, reverse phase high-performance liquid chromatography, and a combination thereof.

12. The method for stabilizing phospholipase according to claim 8, wherein the composition for stabilizing phospholipase increases the stability of the phospholipase after concentration.

13. The method for stabilizing phospholipase according to claim 8, wherein the activity of the phospholipase in the mixed composition after the concentrating step is retained at 80% or more and less than 100% of the activity of the phospholipase of the mixed composition before the concentrating step.

14. The method for stabilizing phospholipase according to claim 8, wherein the phospholipase is derived from a *Trichoderma* sp. microorganism.

15. The method for stabilizing phospholipase according to claim 8, wherein the composition comprising phospholipase comprises a *Trichoderma* sp. microorganism, a culture of the microorganism, a fermentation product of the microorganism, or a combination of two or more thereof.

16. Use of a composition comprising: (i) Tween 20, and (ii) sorbitol, glycerol, or a combination thereof, for stabilizing phospholipase.
